# FASCICULE DE BREVET EUROPEEN

(11) **EP 0 893 978 B1**
(45) Date de publication et mention de la délivrance du brevet: **03.11.1999**
(21) Numéro de dépôt: 97914366.6
(22) Date de dépôt: 11.03.1997
(51) Int. Cl.: A61F 2/46, A61B 17/16, A61F 2/36

(54) **ENSEMBLE DE CHIRURGIE ORTHOPEDIQUE POUR PROTHESE DE HANCHE A COL AMOVIBLE**
SATZ ZUR ORTHOPÄDISCHEN CHIRURGIE FÜR HÜFTPROTHESE MIT ABNEHMBAREM HALS
ORTHOPAEDIC SURGERY ASSEMBLY FOR A HIP PROSTHESIS WITH A REMOVABLE NECK

(30) Priorité: 12.03.1996 FR 9603101
(43) Date de publication de la demande: 03.02.1999
(73) Titulaire: Societe Civile Groupe Grion, 59580 Saint-Saulve (FR)
(72) Inventeur: CHOTEAU, Michel, Pierre, F-59360 Le Cateau (FR); GRYNBLAT, Gérard, Joseph, F-62400 Béthune (FR); LETENDART, Joel, Emile, Roger, F-59880 Saint-Saulve (FR); MATHEVON, Henri, F-59240 Dunkerque (FR); STAHL, Philippe, André, Joseph, F-59262 Sainghin-en-Mélantois (FR); VAN OVERSCHELDE, Jacques Léon Marie Corneille, B-9420 Erpe Mere (BE); VIGIER, Philippe Jean-Marie, Villa Xanteberry, F-64310 Ascain (FR)
(74) Mandataire: Hénnion, Jean-Claude
(86) Numéro de dépôt international: FR9700426
(87) Numéro de publication internationale: WO9733538

(56) Documents cités:
- EP-A- 0 000 549
- EP-A- 0 200 672
- DE-A- 3 147 249
- DE-C- 4 116 507
- FR-A- 2 575 384
- FR-A- 2 580 926
- FR-A- 2 693 367
- FR-A- 2 697 996
- GB-A- 1 371 335
- GB-A- 2 259 859
- US-A- 4 795 469
- US-A- 4 919 679
- US-A- 5 443 471

## Description

La présente invention a pour objet un ensemble de chirurgie orthopédique comportant un ancillaire de prothèse de hanche, une râpe fémorale et une tige fémorale du type à col amovible. Ce col présente une portée conique adaptée pour s'emmancher dans un logement conique conjugué formé dans l'extrémité proximale de la tige. Des tiges de ce type sont décrites notamment par les brevets FR A 8506214 (2580926), 9214120 (2697996) et par le brevet européen EP A 0017743.

Les prothèses de hanche à col amovible sont de plus en plus utilisées en raison des grandes possibilités qu'elles offrent aux chirurgiens pour leur permettre d'adapter chaque prothèse aux besoins spécifiques de leurs patients.

S'il est possible, comme dans le brevet US 4 919 679 qui décrit une prothèse à col fixe, d'utiliser un ancillaire de pose dont l'axe est situé dans le prolongement de la tige de la prothèse et relié à celle-ci par des moyens de liaison tel qu'un filetage, parce que le volume de matériau dans la partie proximale de la tige est suffisant pour permettre le perçage, puis le taraudage d'un trou, ceci n'est plus possible avec des tiges de prothèse destinées à recevoir des cols amovibles. Ceci s'explique notamment par le fait que le perçage du trou de liaison est exécuté en biais par rapport à l'axe de la tige, et interdit de ce fait tout perçage opérationnel orienté dans cet axe.

L'invention concerne une prothèse de hanche à col amovible et porte plus particulièrement sur la liaison de la tige au col ainsi que sur tous les avantages qui en découlent. En effet, une telle prothèse doit comme toute prothèse être aisément manipulée lors de la pose et nécessite l'existence d'un ancillaire spécifique du fait que la prise mécanique par le col n'est plus possible, et qu'il est nécessaire que cette prise soit solide et ferme pour constituer un ensemble compact entre l'ancillaire et la tige. Il en est d'ailleurs de même avec les râpes qui servent à préparer le canal médullaire du fémur à la réception de la tige de prothèse.

C'est pourquoi l'invention porte également non seulement sur une liaison solide et ferme de la tige de prothèse avec son col amovible rendant l'ensemble parfaitement rigide et compact, mais aussi sur un ancillaire spécifique de ce type de prothèse permettant également autant avec la tige de prothèse qu'avec des râpes de réaliser un ensemble parfaitement rigide et compact, de sorte que le chirurgien puisse disposer d'un matériel opératoire fiable, simple et pratique.

Concernant des moyens d'indexation d'une prothèse à col amovible, on connaît par exemple le brevet français A 2 697 996 (MEDINOV) qui prévoit une empreinte d'indexation du col amovible constituée d'une succession de logements semi-circulaires identiques adjacents les uns aux autre sur la périphérie interne du fond du trou de réception. Ces logements sont situés obligatoirement deux à deux en vis à vis et diamétralement opposés pour recevoir une barre transversale de liaison solidaire du col amovible et dont les dimensions correspondent à celles des empreintes. Ces logements semi-circulaires forment ainsi une alternance circonférentielle de concavités et d'arêtes qui fait que lesdites arêtes présentent une fragilité importante non seulement lors de la manipulation du col dans l'empreinte, mais aussi lors des sollicitations mécaniques diverses de la liaison pendant le fonctionnement de la prothèse.

L'invention met à profit le trou réalisé sur la tige pour recevoir le col amovible, pour le prolonger dans son axe pour créer des moyens d'indexation et des moyens de liaison avec un ancillaire.

Des moyens d'extraction éventuelle du col amovible peuvent aussi être prévus pour permettre de nouveau l'accès auxdits moyens de liaison. Il est en effet à prévoir qu'une prothèse puisse un jour être à extraire ou que seulement le col amovible soit à changer en faveur d'un col de dimensions différentes.

Conformément à l'invention, l'ensemble de chirurgie orthopédique comprend des moyens communs de liaison entre l'ancillaire et la tige et entre l'ancillaire et la râpe, permettant la pose par le même ancillaire, dans le canal intramédullaire d'un fémur, aussi bien de la râpe que de la tige fémorale.

Les moyens de liaison comportent, sur la tige, un trou taraudé prolongeant le fond d'un logement de réception du col amovible formé dans la tige, et un doigt fileté constituant une extrémité de l'ancillaire, adapté pour venir se visser dans ledit trou taraudé.

Ces moyens de liaison comportent également, sur la râpe, un trou taraudé prolongeant le fond d'un logement de réception du col amovible, ménagé dans l'extrémité proximale de la râpe, ce trou taraudé étant adapté pour recevoir le doigt fileté de l'ancillaire.

Ainsi, l'invention met à profit le fait que le col de la tige soit amovible, donc que l'extrémité proximale de la tige soit pourvue d'un logement de réception de ce col, pour ménager dans le fond de ce logement un moyen de liaison avec l'ancillaire ; corrélativement l'invention prévoit d'agencer dans l'extrémité proximale de la râpe associée à cette tige, un logement et un moyen de liaison terminal identiques à ceux de la tige.

Avantageusement, l'ancillaire comporte une extrémité désolidarisable en rotation de son manche. En effet, ce dernier est de forme recourbée et ne pourrait donc pas aisément être vissé ou dévissé dans le champ opératoire, lors de l'opération chirurgicale.

Suivant une seconde forme de réalisation de l'invention, le trou taraudé est prolongé par un alésage axial traversant complètement la partie proximale de la tige, cet ensemble comporte un ancillaire d'extraction du col amovible par introduction dans l'alésage et dans le trou taraudé d'une partie terminale de cet ancillaire, et par impaction sur l'extrémité du col amovible, ledit ancillaire étant constitué d'une tige rigide dont la partie terminale a un diamètre légèrement inférieur à celui de l'alésage d'introduction.

L'ancillaire est introduit à travers la corticale du fémur après repérage préalable à l'aide d'un dispositif de visée spécifique.

L'invention procure un avantage appréciable par rapport à la technologie antérieurement utilisée, en termes de coût et de simplification du matériel.

Suivant une autre caractéristique de l'invention, le fond du logement de la râpe et de la tige présente une empreinte profilée dont le bord est constitué par une alternance de courbures concaves et convexes ; une extrémité correspondante du col comporte une partie mâle d'indexation, pourvue d'une alternance complémentaire de courbures convexes et concaves pouvant venir se loger dans les courbures femelles concaves et convexes de l'empreinte, afin d'indexer le col suivant une orientation angulaire déterminée.

D'autres particularités et avantages de l'invention apparaîtront au cours de la description qui va suivre, en référence aux dessins annexés qui en illustrent deux formes de réalisation à titre d'exemples non limitatifs.
La figure 1 est une vue en élévation à échelle réduite d'une tige fémorale du type à col amovible faisant partie de l'ensemble de chirurgie orthopédique conforme à l'invention.
La figure 2 est une vue en coupe transversale, à échelle agrandie, suivant 2-2 de la figure 1.
La figure 3 est une vue en élévation longitudinale du col amovible de la tige des figures 1 et 2.
La figure 4 est une vue en coupe transversale à échelle agrandie de l'extrémité proximale de la tige et des figures 1 et 2, montrant notamment le logement de l'extrémité de l'ancillaire et le moyen de liaison entre celui-ci et la tige.
La figure 5 est une vue en coupe de l'extrémité proximale d'une tige ou d'une râpe, ainsi qu'une vue en élévation partielle de l'ancillaire associé.
La figure 6 est une vue en élévation longitudinale, à échelle réduite, de l'ancillaire faisant partie de l'ensemble selon l'invention.
La figure 7 est une vue en perspective éclatée avec coupe partielle d'une seconde forme de réalisation de la tige fémorale et de l'ancillaire selon l'invention.

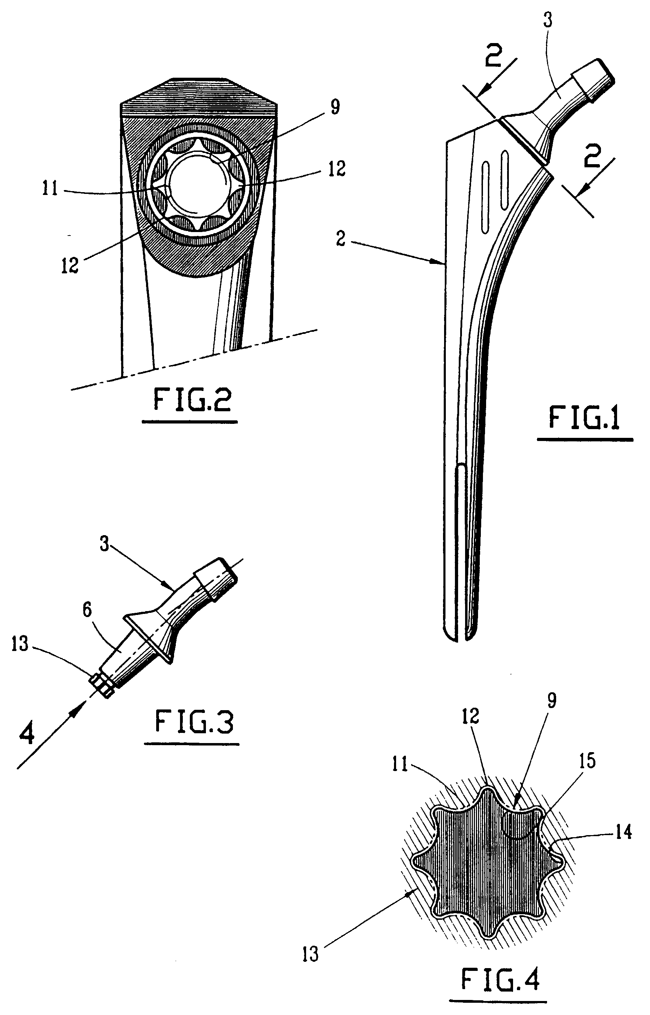

L'ensemble de chirurgie orthopédique représenté aux dessins comprend un ancillaire 1 de prothèse de hanche, une tige fémorale 2 du type à col amovible 3 et une râpe fémorale 10 qui a approximativement le même profil que la tige 2 et est pourvue, dans son extrémité proximale, des mêmes moyens de liaison mécanique avec l'ancillaire 1. Une représentation séparée d'une telle râpe n'est donc pas nécessaire, le dessin de la figure 4 représentant aussi bien l'extrémité proximale d'une tige 2 que d'une râpe 10.

L'ensemble chirurgical comprenant les trois pièces précitées 1, 2 et 10 comprend des moyens de liaison entre l'ancillaire 1 et la tige 2 ou la râpe 10, permettant la pose par le même ancillaire 1, dans le canal intramédullaire d'un fémur, aussi bien de la râpe 10 que de la tige fémorale 2.

Dans l'exemple de réalisation décrit, ces moyens de liaison comportent un trou taraudé 4 agencé dans le fond d'un logement conique 5 de l'extrémité proximale de la tige 2. La surface conique du logement 5 sert de surface d'appui à une portée conique terminale 6 du col 3 ou de l'ancillaire 1 lorsqu'on vient solidariser celui-ci avec la tige 2 dépourvue de son col 3. Corrélativement, l'extrémité de l'ancillaire 1 est munie d'un doigt fileté 7 pouvant venir se visser dans le trou taraudé 4 pour solidariser l'ancillaire 1 et la tige 2.

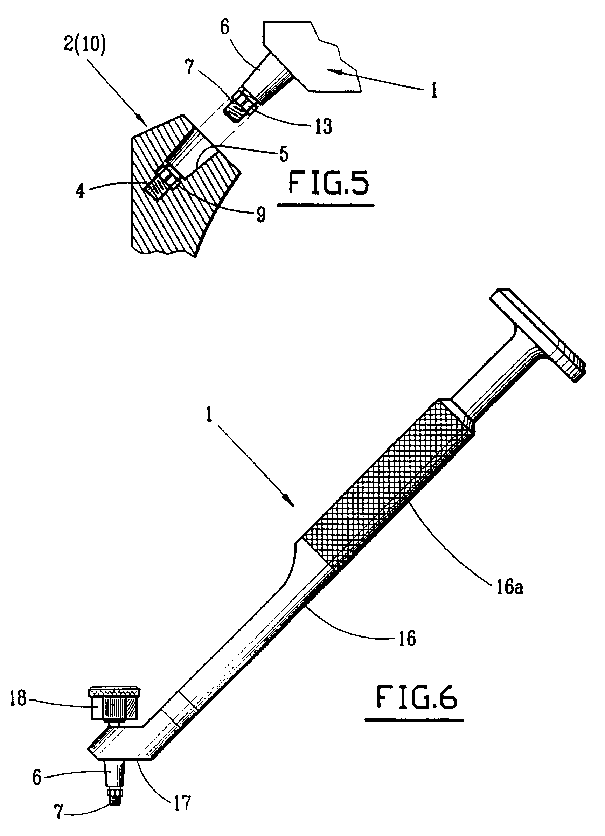

Cette liaison peut être réalisée de la même façon pour solidariser la râpe fémorale 10 (figure 5) et l'ancillaire 1.

Ainsi, le même ancillaire 1 peut, grâce à sa portée conique 6 et à son extrémité filetée 7, venir se solidariser avec, soit la tige 2, soit la râpe fémorale 10, grâce à l'agencement de liaison prévu par l'invention.

Le fond du logement conique 5 de la râpe 10 et de la tige 2 présente une empreinte profilée 9, ménagée sur la périphérie dudit fond et dont le bord est constitué par une alternance de courbures concaves 12 et convexes 11. L'extrémité correspondante du col 3 comporte une partie mâle 13 d'indexation pourvue sur sa périphérie d'une alternance complémentaire de courbures convexes 14 et concaves 15. Ces courbures peuvent venir s'emboîter respectivement dans les courbures femelles concaves 12 et les courbures convexes 11, afin de positionner le col 3 suivant une orientation angulaire déterminée.

Suivant un mode de réalisation avantageux, l'empreinte 9 et la partie mâle d'indexation 13 comprennent respectivement une série de huit courbures concaves 12 et 15 et convexes 11, 14 alternées sur une circonférence, le profil de la partie d'indexation 13 pouvant venir s'emboîter dans l'empreinte 9 suivant une orientation angulaire quelconque.

Les courbures concaves 15 de la partie mâle 13 d'indexation ont des rayons de courbure supérieurs aux rayons de courbure de ses parties convexes 14, intercalées entre les courbures concaves 15. L'alternance des courbures concaves et convexes et leurs différences de rayon de courbure assurent une meilleure répartition des efforts que dans le système du brevet FR-A 2 697 996 grâce à la suppression des arêtes, tout en garantissant une bonne indexation.

Ainsi les parties concaves 15 de l'élément mâle 13 présentent des faces d'appui plus importantes que les parties convexes 14. A titre d'exemple numérique indicatif, pour huit courbures concaves 15 alternées avec huit courbures convexes 14, les rayons de courbure respectifs sont compris avantageusement entre environ 1,40 et 1,60mm et 0,40 à 0,50mm, avec des valeurs préférentielles de 1,55mm et 0,45mm. Ainsi le rapport des rayons des courbures concaves 15 aux rayons des courbures convexes 14 est d'environ 1/3.

Les profils de l'empreinte 9 et de la partie d'indexation angulaire complémentaire 13 présentent une résistance mécanique supérieure à celle de la rosace du FR-A-2697996, grâce à l'alternance de surfaces concaves et convexes et à la suppression corrélative de zones pointues. Cet agencement accroît donc la résistance mécanique de la liaison entre le col amovible et la tige 2 ou la râpe 10. Cet accroissement de résistance est également dû à la présence de deux surfaces alternées concaves et convexes sur l'empreinte 9 et sur la partie d'indexation 13, complémentaires l'une de l'autre, ce qui n'est pas le cas des surfaces correspondantes du FR-A-2697996.

L'ancillaire 1 comprend un manche 16, pourvu d'une zone moletée 16a de préhension manuelle, et par rapport auquel le doigt fileté 7 est désolidarisable en rotation. Plus précisément, le doigt 7 est monté axialement à l'intérieur d'une douille présentant la portée conique 6, librement rotatif et mobile en translation, la douille étant solidaire d'une partie terminale 17 du manche 16 recourbée par rapport au reste du manche.

Le doigt fileté 7 traverse de part en part la partie terminale 17 et est pourvu d'un bouton 18 de préhension manuelle, permettant le vissage du doigt 7 dans le trou taraudé 4.

Grâce à cet agencement le doigt 7 peut être vissé ou dévissé sans devoir faire tourner en même temps le manche 16 dans le champ opératoire, ce qui serait gênant en raison de son extrémité non rectiligne.

Le logement 5 et le trou 4 sont protégés par le col amovible 3, ce qui offre au chirurgien l'avantage de pouvoir y introduire l'extrémité de l'ancillaire 1 après extraction du col amovible 3 sans qu'il soit besoin de mettre en place un bouchon protecteur du volume taraudé 4, comme dans certaines prothèses connues. En effet la présence du col assure cette fonction de protection.

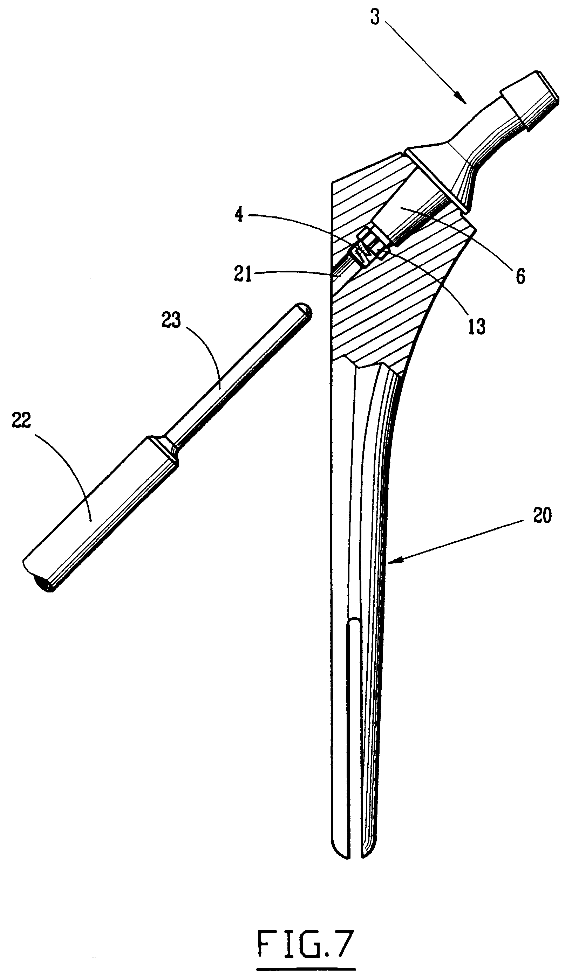

On a représenté à la figure 7 un second mode de réalisation de la tige fémorale 20 : son trou taraudé 4 est prolongé par un alésage axial 21 traversant complètement la partie proximale de la tige 20. A celle-ci est associé un ancillaire 22 constitué d'une tige rigide comportant une partie terminale 23 dont le diamètre est légèrement inférieur à celui de l'alésage 21 d'introduction. Le col 3 peut être extrait du logement 5 et de l'empreinte 9 par impaction sur la partie mâle 13 de l'extrémité de la partie terminale 23 de l'ancillaire 22.

L'alésage 21 peut être obturé par un bouchon (non représenté) en un produit biocompatible tel que le polyéthylène. Ce bouchon peut être extrait par impaction en même temps que le col amovible 3.

## Revendications

1. Ensemble de chirurgie orthopédique comportant un ancillaire (1) de prothèse de hanche, une râpe fémorale (10) et une tige fémorale (2) comprenant des moyens communs de liaison entre l'ancillaire et la tige et entre l'ancillaire et la râpe (10) permettant d'une part la pose de la tige et d'autre part la pose et la manipulation de la râpe, à l'aide du même ancillaire dans le canal intramédulaire d'un fémur, caractérisé en ce que la tige fémorale (2) est équipée d'un col amovible (3) présentant une portée (6) adaptée pour s'emmancher dans un logement (5) conjugué formé dans l'extrémité proximale de la tige, et dont l'extrémité comporte une partie mâle (13) d'indexation pourvue d'une alternance circonférentielle de courbures convexes (14) et concaves (15), et en ce que lesdits moyens communs de liaison comprennent :
- dans l'extrémité proximale de la râpe, un logement de même configuration que celui de la tige fémorale,
- dans le fond du logement (5) de la râpe (10) et de la tige (2), une même empreinte profilée (9) dont le bord (13) est constitué par une alternance circonférentielle de courbures concaves (12) et convexes (11),
- un même trou taraudé prolongeant le fond du logement de la râpe et de la tige,
- et dans l'ancillaire, d'une part une portée et une partie mâle d'indexation identiques à celles du col amovible, et d'autre part un doigt fileté (7) constituant une extrémité dudit ancillaire (1), adapté pour venir se visser dans ledit trou taraudé.

2. Ensemble selon la revendication 1, caractérisé en ce que l'empreinte (9) et la partie mâle d'indexation (13) du col (3) comprennent une série de huit courbures convexes (11, 14) et concaves (12, 15) alternées sur une circonférence.

3. Ensemble selon l'une des revendications 1 ou 2, caractérisé en ce que l'ancillaire (1) comprend un manche (16) par rapport auquel le doigt fileté (7) formant son extrémité est désolidarisable en rotation.

4. Ensemble selon la revendication 3 , caractérisé en ce que le doigt fileté (7) est monté librement rotatif et mobile en translation dans une douille solidaire de la partie terminale (17) du manche (16) et présentant ladite portée (6), et en ce que ce doigt fileté traverse de part en part ladite partie terminale et est pourvu d'un bouton (18) de préhension manuelle.

5. Ensemble selon l'une des revendications 1 à 4, caractérisé en ce que le trou taraudé est prolongé par un alésage axial (21) traversant complètement la partie proximale de la tige (20), et en ce que cet ensemble comporte un ancillaire (22) d'extraction du col amovible (3) par introduction dans l'alésage et dans le trou taraudé (4) d'une partie terminale (23) de cet ancillaire et impaction sur l'extrémité du col amovible ledit ancillaire étant constitué d'une tige rigide dont la partie terminale (23) a un diamètre légèrement inférieur à celui de l'alésage d'introduction (21).

6. Ensemble selon la revendication 5, caractérisé en ce que l'alésage (21) est obturé par un bouchon en un produit biocompatible tel que le polyéthylène.

7. Ensemble selon l'une des revendications 1 à 6 caractérisé en ce que la partie mâle (13) d'indexation du col (3) présente des courbures concaves (15) dont les rayons de courbures sont supérieurs aux rayons de courbure des courbures convexes (14) intercalées entre lesdites courbures concaves.

8. Ensemble selon la revendication 7, caractérisé en ce que pour huit courbures concaves (15) alternées avec huit courbures convexes (14) les rayons de courbure respectifs sont compris entre environ 1,40 et 1,60 mm et 0,40 à 0,50 mm, et en ce que le rapport des rayons des courbures concaves (15) aux rayons des courbures convexes (14) est d'environ 1/3.

## Claims

1. Orthopedic surgery assembly including an ancillary tool (1) for a hip prosthesis, a femoral raspatory(10) and a femoral stem (2) comprising common means of connection between the ancillary tool and the stem and between the ancillary tool and the raspatory (10), permitting on the one hand the positioning of the stem and on the other hand the positioning and manipulation of the raspatory, with the aid of the same ancillary tool in the intramedullary channel of a femur, characterised in that the femoral stem (2) is equipped with a removable neck (3) having a bearing span (6) designed to engage in a matching recess (5) formed in the proximal end of the stem, and of which the end includes a male indexing part (13) provided with circumferential alternating convex (14) and concave (15) curves, and in that the said common means of connection comprise :
- in the proximal end of the raspatory, a recess of the same configuration as that of the femoral stem,
- in the bottom of the recess (5) of the raspatory (10) and of the stem (2), a same profiled indentation (9) whose edge (13) is made up of circumferential alternating concave (12) and convex (11) curves,
- a same tapped hole extending from the bottom of the recess of the raspatory and of the stem,
- and in the ancillary tool, on the one hand a bearing span and a male indexing part identical to those of the removable neck, and on the other hand a threaded projection (7) constituting one end of the said ancillary tool (1), and designed to be screwed into the said tapped hole.

2. Assembly according to claim 1, characterised in that the indentation (9) and the male indexing part (13) of the neck (3) comprise a series of eight convex (11, 14) and concave (12, 15) curves alternating about a circumference.

3. Assembly according to either of claims 1 and 2, characterised in that the ancillary tool (1) comprises a handle (16) from which the threaded projection (7), forming its end, can be detached by rotation.

4. Assembly according to claim 3, characterised in that the threaded projection (7) is mounted free in rotation and movable in translation in a socket integral with the end part (17) of the handle (16) and having said bearing span (6), and in that this threaded projection passes right through said end part and is provided with a knob (18) for gripping manually.

5. Assembly according to one of claims 1 to 4, characterised in that the tapped hole is extended via an axial bore (21) passing completely through the proximal part of the stem (20), and in that this assembly includes an ancillary tool (22) for extracting the removable neck (3) by introduction of an end part (23) of this ancillary tool into the bore and into the tapped hole (4) and by impaction on the end of the removable neck, said ancillary tool consisting of a rigid rod whose end part (23) has a diameter which is slightly smaller than that of the introduction bore (21).

6. Assembly according to claim 5, characterised in that the bore (21) is closed off by a plug made of a biocompatible material such as polyethylene.

7. Assembly according to one of claims 1 to 6, characterised in that the male indexing part (13) of the neck (3) has concave curves (15) whose radii of curvature are greater than the radii of curvature of the convex curves (14) interposed between said concave curves.

8. Assembly according to claim 7, characterised in that for eight concave curves (5) alternating with eight convex curves (14), the respective radii of curvature are between approximately 1.40 and 1.60 mm and 0.40 to 0.50 mm, and in that the ratio of the radii of the concave curves (15) to the radii of the convex curves (14) is approximately 1:3.

## Patentansprüche

1. Set für die orthopädische Chirurgie mit einem Hüftprothesen-Hilfswerkzeug (1), einer Oberschenkelknochen-Reibe (10) und einer Oberschenkelknochen-Stange (2), die gemeinsame Mittel zur Verbindung zwischen dem Hilfswerkzeug und der Stange und zwischen dem Hilfswerkzeug und der Reihe (10) aufweisen, welche einerseits das Anbringen der Stange und andererseits das Anbringen und die Betätigung der Reibe mit Hilfe des gleichen Hilfswerkzeugs im intramedulären Kanal eines Oberschenkelknochens ermöglichen, dadurch gekennzeichnet, daß die Oberschenkelknochen-Stange (2) mit einem lösbaren Hals (3) ausgestattet ist, der einen Zapfen (6) aufweist, welcher sich in einen zugeordneten Sitz (5) einfügen kann, der im nahen Ende der Stange ausgebildet ist, und dessen Ende mit einem indizierten Dorn (13) versehen ist, der an seinem Umfang abwechselnd konvexe (14) und konkave Krümmungen (15) besitzt, und daß die gemeinsamen Verbindungsmittel aufweisen:
- im nahen Ende der Reihe einen Sitz gleicher Konfiguration wie der Sitz der Oberschenkelknochen-Stange,
- am Boden des Sitzes (5) der Reihe (10) und der Stange (2) eine gleiche mit Profil versehene Vertiefung (9), deren Rand aus einer abwechselnden Anordnung von konkaven (12) und konvexen Krümmungen (11) an ihrem Umfang besteht,
- ein gleiches Gewindeloch, das den Boden des Sitzes der Reibe und der Stange verlängert,
- und im Hilfswerkzeug einerseits einen Zapfen und einen indizierten Dorn gleich denen des lösbaren Halses, und andererseits einen mit Gewinde versehene Finger (7), der ein Ende des Hilfswerkzeugs (1) bildet und sich in das Gewindeloch einschraubt.

2. Set nach Anspruch 1, dadurch gekennzeichnet, daß die Vertiefung (9) und der indizierte Dorn (13) des Halses (3) eine Reihe von acht konvexen (11, 14) und konkaven Krümmungen (12, 15) aufweisen, die sich auf einem Umfang abwechseln.

3. Set nach einem der Ansprüche 1 oder 2, dadurch gekennzeichnet, daß das Hilfswerkzeug (1) einen Stiel (16) aufweist, in Bezug auf welchen der mit Gewinde versehene Finger (7), der sein Ende bildet, in Drehrichtung lösbar ist.

4. Set nach Anspruch 3, dadurch gekennzeichnet, daß der mit Gewinde versehene Finger (7) frei drehbar und in Translationsrichtung beweglich in einer Hülse montiert ist, die fest mit dem Endbereich (17) des Stiels (16) verbunden ist und den Zapfen (6) aufweist, und daß dieser mit Gewinde versehene Finger diesen Endbereich von einer Seite zu anderen durchquert und einen Knopf (18) zum manuellen Ergreifen aufweist.

5. Set nach einem der Ansprüche 1 bis 4, dadurch gekennzeichnet, daß das Gewindeloch von einer Axialbohrung (21) verlängert wird, die den nahen Bereich der Stange (20) vollständig durchquert, und daß dieses Set ein Hilfswerkzeug (22) zum Herausziehen des lösbaren Halses (3) durch Einführung eines Endbereichs (23) dieses Hilfswerkzeugs in die Bohrung und in das Gewindeloch (4) und durch Schlagwirkung auf das Ende des lösbaren Halses aufweist, wobei dieses Hilfswerkzeug aus einer steifen Stange besteht, deren Endbereich (23) einen Durchmesser aufweist, der geringfügig kleiner ist als der der Einführungsbohrung (21).

6. Set nach Anspruch 5, dadurch gekennzeichnet, daß die Bohrung (21) von einem Stopfen aus einem biokompatiblen Material wie z.B. Polyethylen verschlossen wird.

7. Set nach einem der Ansprüche 1 bis 6, dadurch gekennzeichnet, daß der Indizierungs-Dorn (13) des Halses (3) konkave Krümmungen (15) aufweist, deren Krümmungsradien größer sind als die Krümmungsradien der konvexen Krümmungen (14), die zwischen den konkaven Krümmungen angeordnet sind.

8. Set nach Anspruch 7, dadurch gekennzeichnet, daß für acht konkave Krümmungen (15), die mit acht konvexen Krümmungen (14) abwechseln, die jeweiligen Krümmungsradien zwischen etwa 1,40 und 1,60 mm und 0,40 bis 0,50 mm liegen, und daß das Verhältnis zwischen den konkaven Krümmungsradien (15) und den konvexen Krümmungsradien (14) etwa 1/3 beträgt.
